# EUROPEAN PATENT SPECIFICATION

(11) **EP 2 752 053 B1**
(45) Date of publication and mention of the grant of the patent: **03.06.2015**
(21) Application number: 12759309.3
(22) Date of filing: 29.08.2012
(51) Int. Cl.: H04W 48/04

(54) **MEMS ACCELEROMETER AIRLINER TAKEOFF AND LANDING DETECTION**
MEMS-BESCHLEUNIGUNGSMESSER EINES LINIENFLUGZEUGS FÜR START- UND LANDEERFASSUNG
DÉTECTION DE DÉCOLLAGE ET D'ATTERRISSAGE D'AVION DE LIGNE À ACCÉLÉROMÈTRE À SYSTÈME MICRO-ÉLECTROMÉCANIQUE (MEMS)

(30) Priority: 01.09.2011 US 201161530328 P; 03.01.2012 US 201213342439
(43) Date of publication of application: 09.07.2014
(73) Proprietor: Qualcomm Incorporated, San Diego, CA 92121-1714 (US)
(72) Inventor: BURKE, John Michael, San Diego, California 92121-1714 (US)
(74) Representative: Catesby, Olivia Joanne
(86) International application number: PCT/US2012/052868
(87) International publication number: WO 2013/033218

(56) References cited:
- WO-A1-97/22079
- US-A1- 2007 042 765
- US-A1- 2009 117 919
- US-A1- 2011 047 112
- LIANG ZHANG ET AL: "A Two-phase Flight Data Feature Selection Method Using both Filter and Wrapper", SOFTWARE ENGINEERING, ARTIFICIAL INTELLIGENCE, NETWORKING, AND PARALLEL/DISTRIBUTED COMPUTING, 2007. SNPD 2007. EIGHTH ACIS INTERNATIONAL CONFERENCE ON, IEEE, PISCATAWAY, NJ, USA, 1 July 2007 (2007-07-01), pages 447-452, XP031124791, ISBN: 978-0-7695-2909-7

## Description

### BACKGROUND

### Field of Disclosure

The present disclosure relates generally to motion event identification, and more specifically to detecting airliner motion events such as takeoff or landing.

### Background

Airliner mobile telephone use is strictly regulated for a number of reasons: interference with sensitive aircraft navigation equipment, potential disruption of electronic aircraft flight control systems, cellular telephony channel reuse assumptions, and talking noise related nuisance to other passengers. There is a general consensus by airlines and government regulators that the risks associated with mobile telephony use on airliners outweigh the potential benefits.

It is therefore desirable to have a simple, accurate, robust, and power efficient mechanism for automatically disabling the mobile telephony features of personal electronic devices immediately after airliners become airborne, and for enabling the same telephony features upon landing.

US 2011/047112A1 discloses a method for detecting airplane flight events using at least one acceleration sensor associated with a mobile communication device includes obtaining at least one acceleration signal from the at least one acceleration sensor; pre-processing the obtained at least one acceleration signal to remove redundant information present in the at least one acceleration signal; performing a feature extraction on the pre-processed at least one acceleration signal; and classifying airplane flight events based on an acceleration pattern represented by the extracted acceleration features.

### SUMMARY

Exemplary embodiments of the invention are directed to systems and methods for identifying mobile device motion events.

In some embodiments, a method is provided for identifying an airliner motion event at a mobile device. The method may comprise, for example: determining scalar acceleration signals from calibrated triaxial accelerometer data obtained from one or more accelerometers; filtering the scalar acceleration signals to reduce high frequency noise; processing the filtered scalar acceleration signals to generate an acceleration spread waveform; comparing the acceleration spread waveform to one or more predetermined patterns characteristic of an airliner motion event; and identifying an airliner motion event based on whether the comparing results in a substantial match.

In other embodiments, an apparatus is provided for identifying an airliner motion event at a mobile device. The apparatus may comprise, for example: means for determining scalar acceleration signals from calibrated triaxial accelerometer data obtained from one or more accelerometers; means for filtering the scalar acceleration signals to reduce high frequency noise; means for processing the filtered scalar acceleration signals to generate an acceleration spread waveform; means for comparing the acceleration spread waveform to one or more predetermined patterns characteristic of an airliner motion event; and means for identifying an airliner motion event based on whether the comparing results in a substantial match.

In still other embodiments, a computer-readable medium comprising code is provided, which, when executed by a processor, causes the processor to perform operations for identifying an airliner motion event at a mobile device. The computer-readable medium may comprise, for example: code for determining scalar acceleration signals from calibrated triaxial accelerometer data obtained from one or more accelerometers; code for filtering the scalar acceleration signals to reduce high frequency noise; code for processing the filtered scalar acceleration signals to generate an acceleration spread waveform; code for comparing the acceleration spread waveform to one or more predetermined patterns characteristic of an airliner motion event; and code for identifying an airliner motion event based on whether the comparing results in a substantial match.

In still other embodiments, a system is provided for identifying an airliner motion event at a mobile device. The system may comprise, for example, one or more processors and memory coupled to the one or more processors. The one or more processors may be configured to: determine scalar acceleration signals from calibrated triaxial accelerometer data obtained from one or more accelerometers, filter the scalar acceleration signals to reduce high frequency noise, process the filtered scalar acceleration signals to generate an acceleration spread waveform, compare the acceleration spread waveform to one or more predetermined patterns characteristic of an airliner motion event, and identify an airliner motion event based on whether the comparing results in a substantial match. The memory may be configured to store related data and/or instructions.

### BRIEF DESCRIPTION OF THE DRAWINGS

The accompanying drawings are presented to aid in the description of embodiments of the invention and are provided solely for illustration of the embodiments and not limitation thereof.
FIG. 1 illustrates a motion event identification device for identifying airliner takeoff and/or landing events according to an example embodiment.
FIG. 2 illustrates an example of a positive acceleration spread event that may be detected within a corresponding positive acceleration spread waveform.
FIG. 3 illustrates an example negative acceleration spread event that may be detected within a corresponding negative acceleration spread waveform.
FIG. 4 illustrates an example acceleration spread pattern that may be used to detect airliner takeoff ground run and climb events.
FIG. 5 illustrates an example acceleration spread pattern that may be used to detect airliner landing ground run events.
FIG. 6 illustrates an example acceleration spread pattern that may be used to detect airliner flight events.
FIG. 7 illustrates a method of identifying airliner motion events, such as takeoff and landing events, from streamed accelerometer data according to an example embodiment.
FIG. 8 illustrates an example wireless communication device that may implement motion event identification according to one or more of the embodiments described herein.

### DETAILED DESCRIPTION

Aspects of the invention are disclosed in the following description and related drawings directed to specific embodiments of the invention. Alternate embodiments may be devised without departing from the scope of the invention. Additionally, well-known elements of the invention will not be described in detail or will be omitted so as not to obscure the relevant details of the invention.

The word "exemplary" is used herein to mean "serving as an example, instance, or illustration." Any embodiment described herein as "exemplary" is not necessarily to be construed as preferred or advantageous over other embodiments. Likewise, the term "embodiments of the invention" does not require that all embodiments of the invention include the discussed feature, advantage or mode of operation.

The terminology used herein is for the purpose of describing particular embodiments only and is not intended to be limiting of embodiments of the invention. As used herein, the singular forms "a," "an," and "the" are intended to include the plural forms as well, unless the context clearly indicates otherwise. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof.

Further, many embodiments are described in terms of sequences of actions to be performed by, for example, elements of a computing device. It will be recognized that various actions described herein can be performed by specific circuits (e.g., application specific integrated circuits (ASICs)), by program instructions being executed by one or more processors, or by a combination of both. Additionally, the sequence of actions described herein can be considered to be embodied entirely within any form of computer readable storage medium having stored therein a corresponding set of computer instructions that upon execution would cause an associated processor to perform the functionality described herein. Thus, the various aspects of the invention may be embodied in a number of different forms, all of which have been contemplated to be within the scope of the claimed subject matter. In addition, for each of the embodiments described herein, the corresponding form of any such embodiments may be described herein as, for example, "logic configured to" perform the described action.

As discussed in the Background above, it is desirable to have a simple, accurate, robust, and power efficient mechanism for automatically controlling the mobile telephony features of personal electronic devices when present on airliners. Accordingly, systems and methods are provided herein for detecting airliner motion events such as takeoff and/or landing using scalar acceleration waveform analysis (e.g., derived from triaxial accelerometer data), and for controlling mobile devices based on these events. Although scalar acceleration takeoff and landing signal levels may be relatively small compared to the always-present gravity of earth, scalar acceleration signals can be filtered for takeoff and landing detection at low bandwidth, reducing noise to acceptable levels.

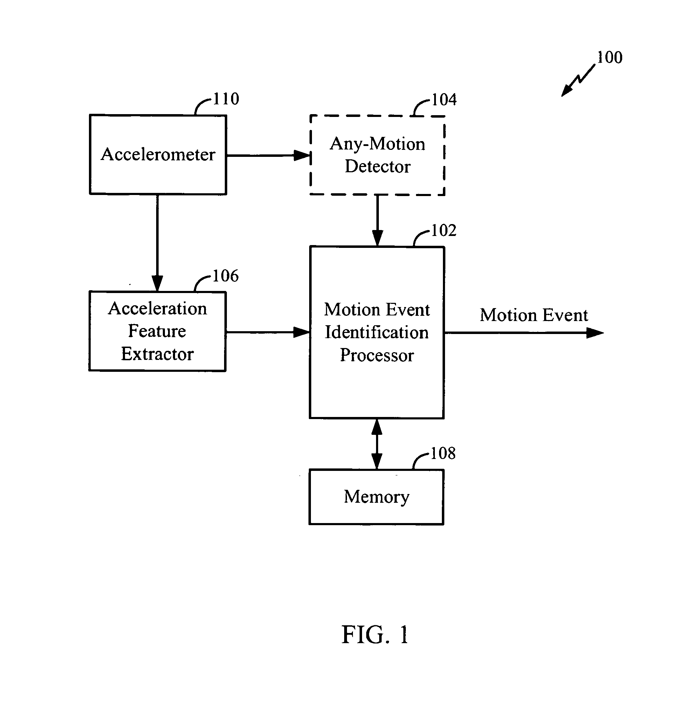

FIG. 1 illustrates a motion event identification device 100 for identifying airliner motion events such as takeoff and/or landing according to an example embodiment. As shown, the motion event identification device 100 includes a motion event identification processor 102 that receives information from an acceleration feature extractor 106. The motion event identification processor 102 is also coupled to a memory 108 configured to store related data and/or instructions. The acceleration feature extractor 106, which may include or be part of one or more processors and/or other hardware components, operates according to acceleration signals received from an accelerometer 110. In some designs, an "any-motion" detector 104 may also be used to detect whether a threshold level of movement is present (i.e., whether the mobile device is "stationary" or not) to obviate the need to perform more complex acceleration feature extraction operations. It will be appreciated that the any-motion detector 104 and acceleration feature extractor 106 may share the accelerometer 110 as shown, or may each have their own accelerometer, either internal or external to the respective detector.

The accelerometer 110 is typically a triaxial accelerometer that provides acceleration data for three different axes. Other examples may include multiple (e.g., three) single axis accelerometers. Further, it will be appreciated that other devices from which acceleration may be derived can be used, so the embodiments should not be construed to be limited to the specific devices discussed explicitly herein.

The acceleration feature extractor 106 monitors the acceleration signals from the accelerometer 110 and performs various processing to characterize acceleration. The acceleration feature extractor 106 may characterize acceleration in a variety of ways, but the characterization typically includes determining a calibrated scalar acceleration signal from the vector acceleration signals (e.g., from triaxial accelerometer data), generating a moving average of the scalar acceleration data over a given averaging period (e.g., a Scalar Acceleration Averaging Period) to filter out high frequency noise, and computing an acceleration spread over a given observation window (e.g., an Acceleration Spread Observation Window Period). Acceleration spread corresponds to the difference between the maximum and the minimum scalar acceleration values in the observation window. The accelerometer 110 may be calibrated, for example, to the extent that any modification of device orientation generates less than a one percent change in acceleration spread. For some applications, it has been found that a Scalar Acceleration Averaging Period on the order of ten seconds (e.g., about twelve seconds, or anywhere in the range of about one and about sixty seconds) and an Acceleration Spread Observation Window Period on the order of a minute or so (e.g., about sixty seconds, or anywhere in the range of about ten and about one hundred and eighty seconds) provide acceleration spread waveforms that yield good results for airliner motion event identification. It will be appreciated, however, that other moving average periods and observation windows may be used as desired, depending on the particular application.

The acceleration feature extractor 106 outputs the extracted acceleration feature information (e.g., an acceleration spread waveform), and provides this information to the motion event identification processor 102. The motion event identification processor 102 is accordingly configured to determine whether the extracted acceleration features correspond to the takeoff or landing of an airliner, such as by employing various pattern matching techniques to recognize acceleration spread events, or a combination thereof, that are unique to airliner takeoffs and landings, as distinguished from other motion events (e.g., pedestrial stepping, automobile acceleration, etc.).

In general, acceleration spread events may be "positive" or "negative," depending on the sign of the acceleration spread during the event. For example, the acceleration spread may be deemed positive if the observation window minimum occurs before the maximum, and negative otherwise.

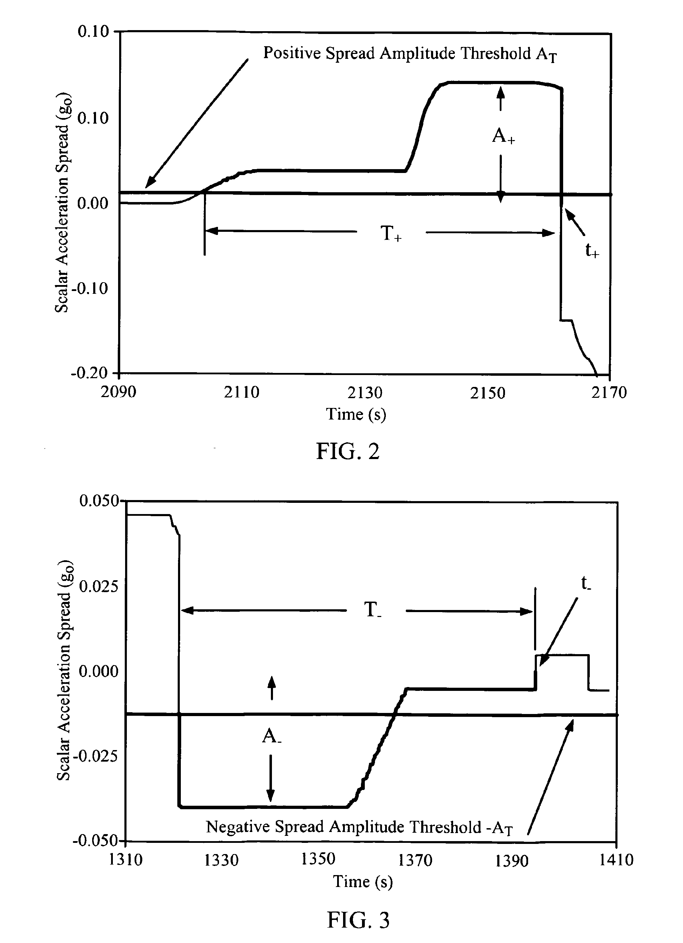

FIG. 2 illustrates an example of a positive acceleration spread event that may be detected within a corresponding positive acceleration spread waveform. As shown, the positive acceleration spread event may be characterized by a positive spread event period T₊ and a positive spread event amplitude A₊. The positive spread event period T₊ may be defined as the time difference between when the acceleration spread amplitude attains a value greater than a given amplitude threshold A_{T} and when it decreases to a value equal to zero, for example. For some applications, it has been found that an amplitude threshold on the order of a few hundredths of the acceleration due to gravity (e.g., an A_{T} of about 0.0125 gₒ) is sufficient to distinguish a positive spread event from background noise or the like. The positive spread event amplitude A₊ may be defined as the maximum acceleration spread amplitude value attained during the positive spread event. The instant t₊ that the acceleration spread amplitude becomes equal to zero after the beginning of the positive spread event may also be defined for the positive spread event in some designs.

FIG. 3 illustrates an example negative acceleration spread event that may be detected within a corresponding negative acceleration spread waveform. As shown, the negative acceleration spread event may be characterized by a negative spread event period T₋ and a negative spread event amplitude A₋. The negative spread event period T₋ may be defined as the time difference between when the acceleration spread amplitude attains a value less than a given amplitude threshold -A_{T} and when it increases to a value equal to zero, for example. For some applications, it has been found that an amplitude threshold on the order of a few hundredths of the acceleration due to gravity (e.g., a -A_{T} of about 0.0125 gₒ) is sufficient to distinguish a negative spread event from background noise or the like. The negative spread event amplitude A₋ may be defined as the minimum acceleration spread amplitude value attained during the negative spread event. The instant t₋ that the acceleration spread amplitude becomes equal to zero after the beginning of the negative spread event may also be defined for the negative spread event in some designs.

The motion event identification processor 102 may implement various algorithms to identify airliner motion events such as takeoff and landing based on the detection of one or more acceleration spread events, including basic as well as enhanced identification algorithms. For example, basic detection algorithms may look for the acceleration patterns characteristic of airliner takeoff ground run and climb events or landing ground run events and their associated quiescent periods. Enhanced algorithms may further require a certain number of "flight events" to be detected within a specified period prior to landing events and subsequent to takeoff events.

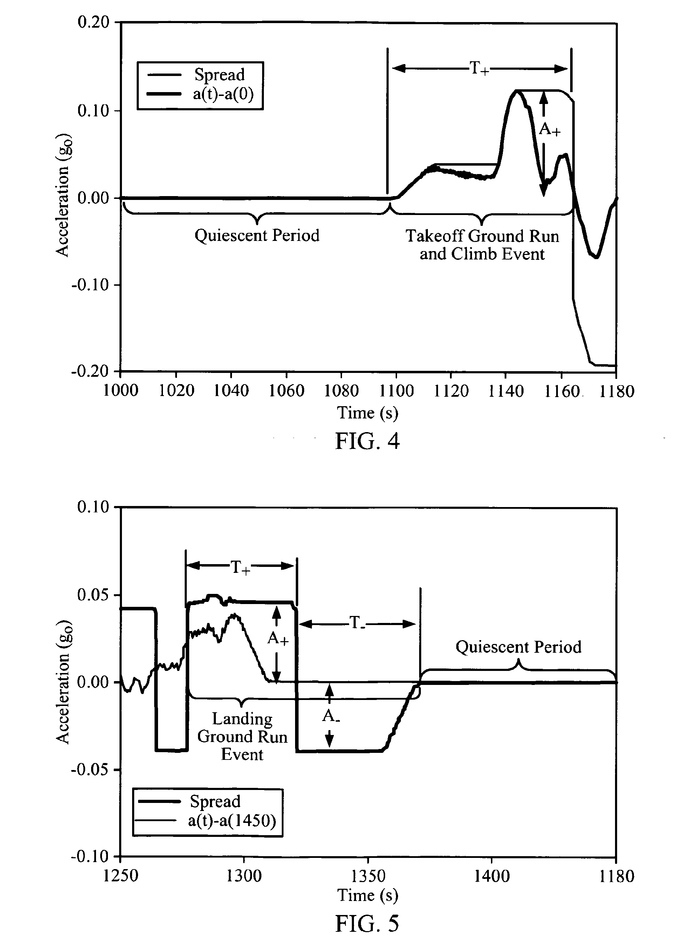

FIG. 4 illustrates an example acceleration spread pattern that may be used to detect airliner takeoff ground run and climb events. As shown, airliner takeoff ground run and climb events may be characterized by a positive spread event with a positive event period T₊ within a specified range and a positive event amplitude A₊ within a specified range. The positive event period range specified for detecting an airliner takeoff ground run and climb event may be defined by a Minimum Takeoff Ground Run and Climb Event Period (T_{Min}) and a Maximum Takeoff Ground Run and Climb Event Period (T_{Max}). Similarly, the positive event amplitude range specified for detecting an airliner takeoff ground run and climb event may be defined by a Minimum Takeoff Ground Run and Climb Event Amplitude (A_{Min}). The motion event identification processor 102 may accordingly identify an airliner takeoff ground run and climb event when it detects a positive spread event having a positive event period T₊ that falls between T_{Min} and T_{Max} with a positive event amplitude A₊ that meets or exceeds A_{Min}.

For some applications, it has been found that a Takeoff Ground Run and Climb Event Period on the order of tens of seconds (e.g., a T_{Min} of about 30 seconds and a T_{Max} of about 100 seconds) used in conjunction with a Minimum Takeoff Ground Run and Climb Event Amplitude on the order of a tenth of the acceleration due to gravity (e.g., an A_{Min} of about 0.075 gₒ) yields appropriate airliner takeoff ground run and climb event detection results. It will be appreciated; however, that other takeoff ground run and climb event periods and/or minimum amplitudes may be used as desired, depending on the particular application.

The motion event identification processor 102 may accordingly identify an airliner takeoff event when it detects a takeoff ground run and climb event acceleration spread pattern. However, airliner takeoff ground run and climb events typically follow a quiescent period, corresponding to periods of relatively little scalar acceleration change as the airliner is taxied to the end of the runway prior to takeoff and subsequently waits for permission to begin the takeoff ground run. Accordingly, in some designs, the motion event identification processor 102 may identify an airliner takeoff event when it detects a takeoff ground run and climb event acceleration spread pattern that follows a quiescent period of minimum duration as shown in FIG. 4. That is, the motion event identification processor 102 may refrain from declaring an airliner takeoff event based on what otherwise would appear to be a takeoff ground run and climb event unless it follows a Minimum Takeoff Event Quiescent Period (ΔT_{T}). For some applications, it has been found that a Minimum Takeoff Event Quiescent Period on the order of a few minutes (e.g., a ΔT_{T} of about 300 seconds) provides sufficient certainty as to whether a subsequently detected takeoff ground run and climb event does in fact correspond to an airliner takeoff event. It will be appreciated, however, that other quiescent period minimum durations may be used as desired, depending on the particular application.

FIG. 5 illustrates an example acceleration spread pattern that may be used to detect airliner landing ground run events. As shown, airliner landing ground run events may be characterized by adjacent positive and negative spread events with a positive event period T₊ within a specified range, a positive event amplitude A₊ within a specified range, a negative event period T₋ within a specified range, and a negative event amplitude A₋ within a specified range. The positive event period range specified for detecting an airliner landing ground run event may be defined by a Minimum Landing Positive Ground Run Event Period (T_{+Min}) and a Maximum Landing Positive Ground Run Event Period (T_{+Max}), while the positive event amplitude range specified for detecting an airliner landing ground run event may be defined by a Minimum Landing Positive Ground Run Event Amplitude (A_{+Min}) and a Maximum Landing Positive Ground Run Event Amplitude (A_{+Max}). Similarly, the negative event period range specified for detecting an airliner landing ground run event may be defined by a Minimum Landing Negative Ground Run Event Period (T_{-Min}) and a Maximum Landing Negative Ground Run Event Period (T_{-Max}), while the negative event amplitude range specified for detecting an airliner landing ground run event may be defined by a Minimum Landing Negative Ground Run Event Amplitude (A_{-Min}) and a Maximum Landing Negative Ground Run Event Amplitude (A_{-Max}). The motion event identification processor 102 may accordingly identify an airliner landing ground run event when it detects one or more adjacent positive and negative spread events, the positive spread events having a positive event period T₊ that falls between T_{+Min} and T_{+Max} with a positive event amplitude A₊ that falls between A_{+Min} and A_{+Max}, and the negative spread events having a negative event period T₋ that falls between T_{-Min} and T_{-Max} with a negative event amplitude A₋ that falls between A_{-Min} and A_{-Max}.

For some applications, it has been found that a Landing Positive Ground Run Event Period on the order of several tens of seconds (e.g., a T_{+Min} of about 35 seconds and a T_{+Max} of about 85 seconds) used in conjunction with a Landing Positive Ground Run Event Amplitude Range on the order of a few to several hundredths of the acceleration due to gravity (e.g., an A_{+Min} of about 0.036 gₒ and an A_{+Max} of about 0.070 gₒ), and a Landing Negative Ground Run Event Period on the order of tens of seconds to a few minutes (e.g., a T_{-Min} of about 35 seconds and a T_{-Max} of about 120 seconds) used in conjunction with a Landing Negative Ground Run Event Amplitude Range on the order of a few to several hundredths of the acceleration due to gravity (e.g., an A_{-Min} of about -0.036 gₒ and an A_{-Max} of about -0.070 gₒ), yield appropriate airliner landing ground run detection results. It will be appreciated, however, that other landing ground run event periods and/or amplitude ranges may be used as desired, depending on the particular application.

The motion event identification processor 102 may accordingly identify an airliner landing event when it detects a landing ground run event acceleration spread pattern. However, airliner landing ground run events are typically followed by a quiescent period, corresponding to periods of relatively little scalar acceleration change as the airliner is taxied from the runway to its gate and waits to be prepared for passengers to unload. Accordingly, in some designs, the motion event identification processor 102 may identify an airliner landing event when it detects a landing ground run event acceleration spread pattern that is followed by a quiescent period of minimum duration as shown in FIG. 5. That is, the motion event identification processor 102 may refrain from declaring an airliner landing event based on what otherwise would appear to be a landing ground run event unless it is followed by a Minimum Landing Event Quiescent Period (ΔT_{L}). For some applications, it has been found that a Minimum Landing Event Quiescent Period on the order of one to a few minutes (e.g., a ΔT_{L} of about 90 seconds) provides sufficient certainty as to whether a previously detected landing ground run event does in fact correspond to an airliner landing event. It will be appreciated, however, that other quiescent period minimum durations may be used as desired, depending on the particular application.

As discussed above, enhanced algorithms may further include the detection and use of certain flight events to verify the integrity of any otherwise identified airliner motion events. Such flight events may be detected, for example, from acceleration spread patterns associated with low frequency, large amplitude scalar acceleration changes typical of in-flight environments where the airliner's acceleration vector is generally aligned with that of gravity.

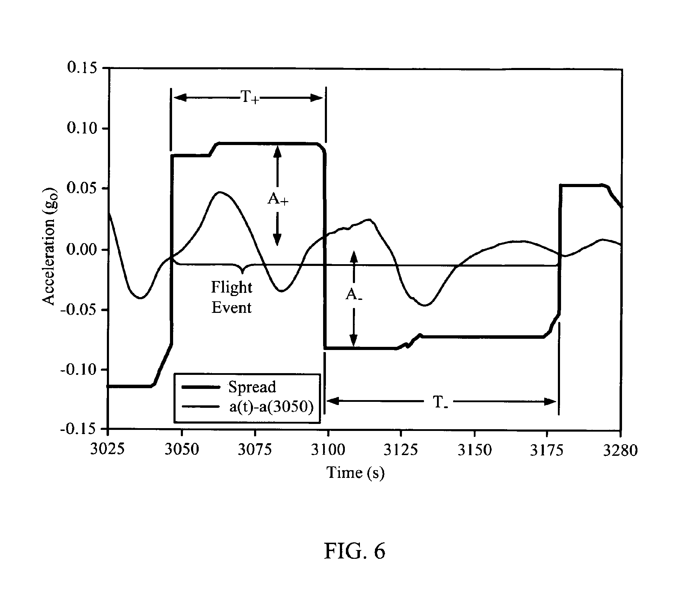

FIG. 6 illustrates an example acceleration spread pattern that may be used to detect airliner flight events. As shown, airliner flight events may be characterized by adjacent positive and negative spread events with a positive event period T₊ within a specified range, a positive event amplitude A₊ within a specified range, a negative event period T₋ within a specified range, and a negative event amplitude A₋ within a specified range. The positive event period range specified for detecting an airliner flight event may be defined by a Minimum Positive Flight Event Period (T_{+Min}) and a Maximum Positive Flight Event Period (T_{+Max}), while the positive event amplitude range specified for detecting an airliner flight event may be defined by a Minimum Positive Flight Event Amplitude (A_{+Min}) and a Maximum Positive Flight Event Amplitude (A_{+Max}). Similarly, the negative event period range specified for detecting an airliner flight event may be defined by a Minimum Negative Flight Event Period (T_{-Min}) and a Maximum Negative Flight Event Period (T_{-Max}), while the negative event amplitude range specified for detecting an airliner flight event may be defined by a Minimum Negative Flight Event Amplitude (A_{-Min}) and a Maximum Negative Flight Event Amplitude (A_{-Max}). The motion event identification processor 102 may accordingly identify an airliner flight event when it detects one or more adjacent positive and negative spread events, the positive spread events having a positive event period T₊ that falls between T_{+Min} and T_{+Max} with a positive event amplitude A₊ that falls between A_{+Min} and A_{+Max}, and the negative spread events having a negative event period T₋ that falls between T_{-Min} and T_{-Max} with a negative event amplitude A₋ that falls between A_{-Min} and A_{-Max}.

For some applications, it has been found that a Positive Flight Event Period on the order of tens of seconds to a few minutes (e.g., a T_{+Min} of about 15 seconds and a T_{+Max} of about 150 seconds) used in conjunction with a Positive Flight Event Amplitude Range on the order of a few tenths of the acceleration due to gravity (e.g., an A_{+Min} of about 0.045 gₒ and an A_{+Max} of about 0.300 gₒ), and a Negative Flight Event Period on the order of tens of seconds to a few minutes (e.g., a T_{-Min} of about 15 seconds and a T_{-Max} of about 150 seconds) used in conjunction with a Negative Flight Event Amplitude Range on the order of a few tenths of the acceleration due to gravity (e.g., an A_{-Min} of about -0.045 gₒ and an A_{-Max} of about -0.300 gₒ), yield appropriate airliner flight detection results. It will be appreciated, however, that other flight event periods and/or amplitude ranges may be used as desired, depending on the particular application.

The motion event identification processor 102 may accordingly identify an airliner flight event when it detects a flight event acceleration spread pattern such as the one illustrated in FIG. 6. Accordingly, in some designs, the motion event identification processor 102 may use the detected flight event information to supplement the acceleration patterns characteristic of airliner takeoff ground run and climb events or landing ground run events, and their associated quiescent periods, as described above, in distinguishing airliner takeoff and landing events from other motion events.

For example, the motion event identification processor 102 may refrain from declaring an airliner takeoff event based on what would otherwise appear to be one unless it is followed by a Minimum Number of Takeoff Flight Events (N_{FET}) over a Maximum Takeoff Flight Event Period (T_{FET}) following the takeoff ground run and climb acceleration spread pattern event. Similarly, the motion event identification processor 102 may refrain from declaring an airliner landing event based on what would otherwise appear to be one unless it is preceded by a Minimum Number of Landing Flight Events (N_{FEL}) over a Maximum Landing Flight Event Period (T_{FEL}) before the landing acceleration spread pattern event. For some applications, it has been found that a Minimum Number of Takeoff Flight Events on the order of one to several (e.g., an N_{FET} of about 2) in conjunction with a Maximum Takeoff Flight Event Period on the order of a few minutes (e.g., a T_{FET} of about 300 seconds), and a Minimum Number of Landing Flight Events on the order of one to several (e.g., an N_{FEL} of about 2) in conjunction with a Maximum Landing Flight Event Period on the order of several minutes (e.g., a T_{FEL} of about 480 seconds), provide sufficient certainty as to whether a given airliner takeoff ground run and climb event or landing ground run event, and their associated quiescent periods, does in fact correspond to an airliner motion event. It will be appreciated, however, that other flight event counts and periods may be used as desired, depending on the particular application.

The identification of airliner motion events such as takeoff and landing may be useful in a variety of post-identification activities. For example, airliner motion event identification may be used to facilitate automatically disabling the mobile telephony features of a mobile device onboard the airliner (e.g., when the mobile device is in the user's front seat pocket, carry-on luggage, stowed baggage, etc.). This is often referred to as entering "airplane mode," whereby the user is allowed to listen to music, play a game, or review stored email messages, for example, but is prevented from making phone calls, use Bluetooth accessories, etc. Usually, only mobile station wireless communication features are rendered inoperative in airplane mode. Accordingly, the motion event identification processor 102 may be further configured to cause a corresponding mobile device (not shown) to enter airplane mode or the like during flight (e.g., after identification of an airliner takeoff event) and/or to exit airplane mode or the like following completion of the flight (e.g., after identification of an airliner landing event).

In addition, the motion event identification processor 102 may be further configured to provide, to the user or another device, certain contextual information or other services related to travel in a commercial airliner. For example, the motion event identification processor 102 may be further configured to cause a corresponding mobile device (not shown) to display flight progress or timing information, to automatically disable speakerphone functionality, or to provide updated connecting flight information.

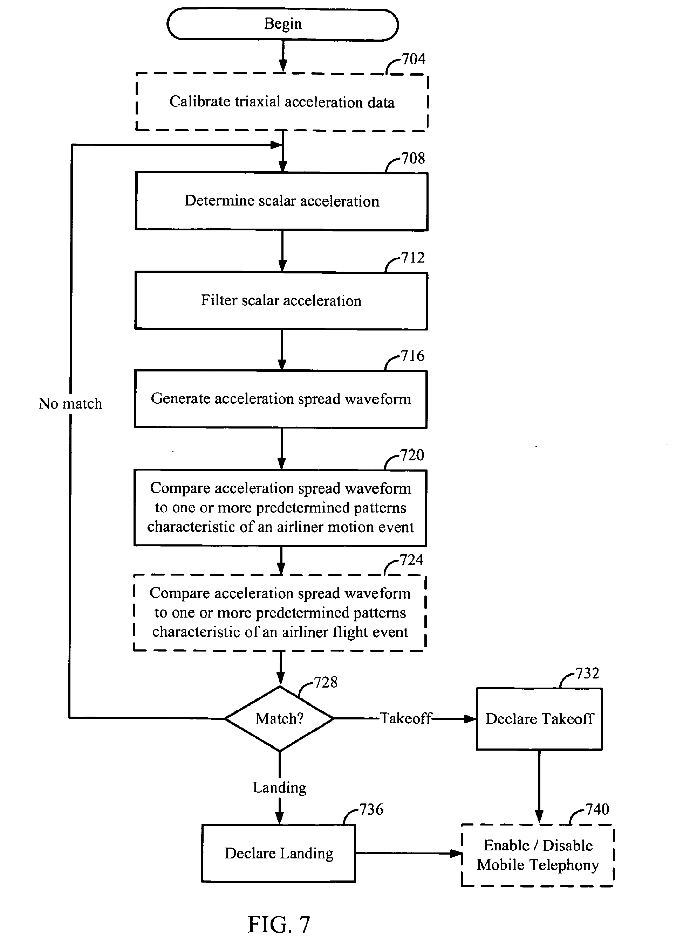

FIG. 7 illustrates a method of identifying airliner motion events, such as takeoff and landing events, from streamed accelerometer data according to an example embodiment. The example method may be performed by one or more processors in conjunction with memory (e.g., the acceleration feature extractor 106, motion event identification processor 102, and memory 108), or various other means as described herein.

As shown, accelerometer measurement error may be initially determined and calibrated to required standards to ensure that inferred scalar acceleration is orientation independent to an appropriate degree (block 704). For example, the one or more accelerometers may be calibrated to the extent that any modification of device orientation generates less than a one percent change in acceleration spread. Calibrated triaxial acceleration data is then obtained, at a particular sampling frequency, and the scalar acceleration signal is computed (block 708). The scalar acceleration signal may be filtered at low bandwidth to reduce noise (block 712), and the acceleration spread waveform may be generated for an optimized window period (block 716).

The acceleration spread waveform may then be compared to one or more predetermined patterns characteristic of an airliner motion event (block 720), and in some embodiments, to one or more predetermined patterns characteristic of an airliner flight event (block 724), as discussed above. An airliner motion event may then be identified (decision 728) as a takeoff event when the comparing results in a substantial match with a takeoff pattern (block 732), or a landing event when the comparing results in a substantial match with a landing pattern (block 736). If no match is found, operation may return to determining the scalar acceleration signal over a subsequent time window (block 708). In some embodiments, where flight events are taken into account, the identification of the airliner motion event may be further based on whether a minimum number of flight events are detected over a given time period.

As discussed in more detail above, the one or more predetermined patterns may include an acceleration spread pattern having a quiescent period followed by an airliner takeoff ground run and climb event. The airliner takeoff ground run and climb event may be defined, for example, by a positive acceleration spread having an amplitude above a specified threshold for a time period within a specified range. The one or more predetermined patterns may also include an acceleration spread pattern having a landing ground run event followed by a quiescent period. The landing ground run event may be defined, for example, by a positive acceleration spread having an amplitude within a specified range for a time period within a specified range, and a negative acceleration spread, adjacent in time to the positive acceleration spread, having an amplitude within a specified range for a time period within a specified range.

The use of the acceleration spread in the manner described above advantageously provides for an efficient identification of airliner motion events, such as airliner takeoff and landing events. In some embodiments, the airliner motion event identification may be used to enable or disable at least one telephony function of the mobile device (block 740).

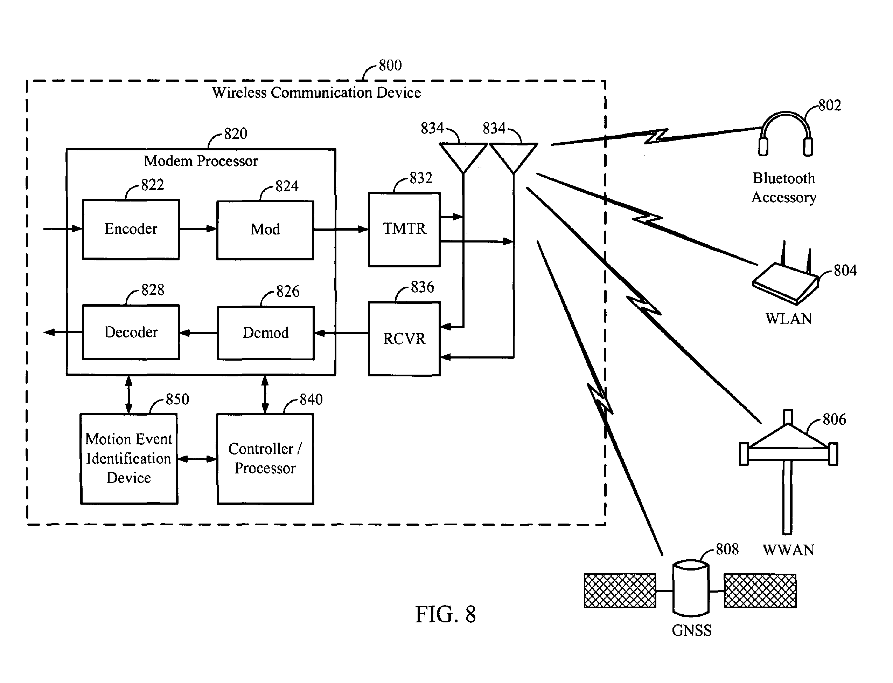

FIG. 8 illustrates an example wireless communication device that may implement motion event identification according to one or more of the embodiments described above. As shown, the wireless communication device 800 may be capable of communicating with various accessory devices 802, local area networks 804, wide area networks 806, satellite systems 808, etc., using built-in or external hardware. For example, on the transmit path, traffic data to be sent by wireless communication device 800 may be processed (e.g., formatted, encoded, and interleaved) by an encoder 822 and further processed (e.g., modulated, channelized, and scrambled) by a modulator (Mod) 824 in accordance with an applicable radio technology (e.g., for Wi-Fi or WWAN) to generate output chips. A transmitter (TMTR) 832 may then condition (e.g., convert to analog, filter, amplify, and upconvert) the output chips and generate a modulated signal, which is transmitted via one or more antennas 834.

On the receive path, antennas 834 may receive signals transmitted by mobile accessory devices 802 (e.g., Bluetooth), various access points 804 (e.g., WLAN) or 806 (e.g., WWAN), Global Navigation Satellite Systems (GNSS) 808 (e.g., GPS), etc. A receiver (RCVR) 836 may then condition (e.g., filter, amplify, downconvert, and digitize) a received signal from the one or more antennas 834 and provide samples. A demodulator (Demod) 826 may process (e.g., descramble, channelize, and demodulate) the samples and provide symbol estimates. A decoder 828 may further process (e.g., deinterleave and decode) the symbol estimates and provide decoded data. Encoder 822, modulator 824, demodulator 826, and decoder 828 may comprise a modem processor 820. These units may perform processing in accordance with the radio technology or technologies used for communication.

According to one or more embodiments, the wireless communication device 800 may further include a motion event identification device 850 of the type described above, such as the motion event identification device 100 shown in FIG. 1. The motion event identification device 850, acting on its own or through a controller/processor 840, may act to automatically disable the operation of one, some, or all of the illustrated mobile telephony components when an airline takeoff event is identified (e.g., cause the wireless communication device 800 to enter airplane mode), and automatically re-enable those components when an airline landing event is identified (e.g., cause the device 800 to exit airplane mode). In addition or alternatively, the motion event identification device 850 may provide certain contextual information related to travel in a commercial airliner to provide enhanced user information and services.

Those of skill in the art will appreciate that information and signals may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Further, those of skill in the art will appreciate that the various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

The methods, sequences and/or algorithms described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor.

Accordingly, an embodiment of the invention can include a computer readable media embodying a method for identifying an airliner takeoff or landing event at a mobile device using triaxial accelerometer data. Accordingly, the invention is not limited to illustrated examples and any means for performing the functionality described herein are included in embodiments of the invention.

While the foregoing disclosure shows illustrative embodiments of the invention, it should be noted that various changes and modifications could be made herein without departing from the scope of the invention as defined by the appended claims. The functions, steps and/or actions of the method claims in accordance with the embodiments of the invention described herein need not be performed in any particular order. Furthermore, although elements of the invention may be described or claimed in the singular, the plural is contemplated unless limitation to the singular is explicitly stated.

## Claims

1. A method of identifying an airliner motion event at a mobile device, comprising:
determining (708) scalar acceleration signals from calibrated triaxial accelerometer data obtained from one or more accelerometers;
filtering (712) the scalar acceleration signals to reduce high frequency noise;
processing the filtered scalar acceleration signals to generate (716) an acceleration spread waveform, wherein the acceleration spread waveform tracks a difference between a maximum value and a minimum value of the filtered scalar acceleration signals over an observation window;
comparing (720) the acceleration spread waveform to one or more predetermined patterns characteristic of an airliner motion event; and
identifying an airliner motion event based on whether the comparing results in a substantial match (728).

2. The method of claim 1, wherein the airliner motion event is a takeoff event or a landing event.

3. The method of claim 1, wherein the observation window has a period of between about ten and about one hundred and eighty seconds.

4. The method of claim 1, wherein the determining comprises averaging the calibrated triaxial accelerometer data over an averaging period of between about one and about sixty seconds.

5. The method of claim 1, wherein the one or more predetermined patterns include an acceleration spread pattern having a quiescent period followed by an airliner takeoff ground run and climb event.

6. The method of claim 5, wherein the airliner takeoff ground run and climb event is defined by a positive acceleration spread having an amplitude above a specified threshold for a time period within a specified range.

7. The method of claim 1, wherein the one or more predetermined patterns include an acceleration spread pattern having a landing ground run event followed by a quiescent period.

8. The method of claim 7, wherein the landing ground run event is defined by a positive acceleration spread having an amplitude within a specified range for a time period within a specified range, and a negative acceleration spread, adjacent in time to the positive acceleration spread, having an amplitude within a specified range for a time period within a specified range.

9. The method of claim 1, further comprising comparing the acceleration spread waveform to one or more predetermined patterns characteristic of an airliner flight event, wherein identifying the airliner motion event is further based on whether a minimum number of flight events are detected over a given time period.

10. The method of claim 1, further comprising enabling or disabling at least one telephony function of the mobile device based on the airliner motion event identification.

11. The method of claim 1, wherein the one or more accelerometers are calibrated to the extent that any modification of device orientation generates less than a one percent change in acceleration spread.

12. An apparatus for identifying an airliner motion event at a mobile device, comprising:
means for determining scalar acceleration signals from calibrated triaxial accelerometer data obtained from one or more accelerometers;
means for filtering the scalar acceleration signals to reduce high frequency noise
means for processing the filtered scalar acceleration signals to generate an acceleration spread waveform, wherein the acceleration spread waveform tracks a difference between a maximum value and a minimum value of the filtered scalar acceleration signals over an observation window;
means for comparing the acceleration spread waveform to one or more predetermined patterns characteristic of an airliner motion event; and
means for identifying an airliner motion event based on whether the comparing results in a substantial match.

13. The apparatus of claim 12 further comprising:
one or more accelerometers (110) configured to output calibrated triaxial accelerometer data; and
wherein the means for determining scalar acceleration signals, the means for filtering, and the means for processing
comprises an acceleration feature extractor (106); and wherein the means for comparing and means for identifying comprisesa motion event identification processor (102).

14. A computer-readable medium comprising code, which, when executed by a processor, causes the processor to perform the method of any of claims 1 to 11.

15. A system for identifying an airliner motion event at a mobile device, comprising:
one or more processors configured to:
determine scalar acceleration signals from calibrated triaxial accelerometer data obtained from one or more accelerometers,
filter the scalar acceleration signals to reduce high frequency noise,
process the filtered scalar acceleration signals to generate an acceleration spread waveform, wherein the acceleration spread waveform tracks a difference between a maximum value and a minimum value of the filtered scalar acceleration signals over an observation window,
compare the acceleration spread waveform to one or more predetermined patterns characteristic of an airliner motion event, and
identify an airliner motion event based on whether the comparing results in a substantial match; and
memory (108) coupled to the one or more processors and configured to store related data and/or instructions.

## Patentansprüche

1. Ein Verfahren zum Identifizieren eines Verkehrsflugzeugbewegungsvorganges an einer Mobileirichtung, das Folgendes aufweist:
Bestimmen (708) von skalaren Beschleunigungssignalen aus kalibrierten, triaxialen Beschleunigungsmesserdaten, die von einem oder mehrere Beschleunigungsmessern erhalten werden;
Filtern (712) der skalaren Beschleunigungssignale, um Hochfrequenzrauschen zu verringern;
Verarbeiten der gefilterten, skalaren Beschleunigungssignale, um eine Beschleunigungsspannenwellenform zu generieren (716), wobei die Beschleunigungsspannenwellenform eine Differenz zwischen einem maximalen Wert und einem minimalen Wert der gefilterten, skalaren Beschleunigungssignale über ein Beobachtungsfenster hinweg nachverfolgt;
Vergleichen (720) der Beschleunigungsspannenwellenform mit einem oder mehreren vorbestimmten Mustern, die für einen Verkehrsflugzeugbewegungsvorgang charakteristisch sind; und
Identifizieren eines Verkehrsflugzeugbewegungsvorganges basierend darauf, ob das Vergleichen zu einer wesentlichen Übereinstimmung (728) führt.

2. Verfahren nach Anspruch 1, wobei der Verkehrsflugzeugbewegungsvorgang ein Abflug- bzw. Startvorgang oder ein Landevorgang ist.

3. Verfahren nach Anspruch 1, wobei das Beobachtungsfenster eine Dauer zwischen ungefähr zehn und ungefähr einhundertachtzig Sekunden hat.

4. Verfahren nach Anspruch 1, wobei das Bestimmen Mitteln der kalibrierten, triaxialen Beschleunigungsmesserdaten über eine Mittelungsdauer zwischen ungefähr einer und ungefähr sechzig Sekunden aufweist.

5. Verfahren nach Anspruch 1, wobei das eine oder die mehreren vorbestimmten Muster ein Beschleunigungsspannenmuster beinhalten, das eine Ruheperiode hat, der ein Verkehrsflugzeugabflugbodenfahrt- und -steigflugvorgang folgt.

6. Verfahren nach Anspruch 5, wobei der Verkehrsflugzeugabflugbodenfahrt- und -steigflugvorgang definiert ist durch eine positive Beschleunigungsspanen mit einer Amplitude über einem bestimmten Schwellenwert für eine Zeitdauer innerhalb eines spezifizierten Bereichs.

7. Verfahren nach Anspruch 1, wobei das eine oder die mehreren vorbestimmten Muster ein Beschleunigungsspannenmuster mit einem Landebodenfahrtvorgang, dem eine Ruheperiode folgt, beinhaltet

8. Verfahren nach Anspruch 7, wobei der Landebodenfahrtvorgang definiert wird durch eine positive Beschleunigungsspanne mit einer Amplitude innerhalb eines spezifizierten Bereiches für eine Zeitdauer innerhalb eines spezifizierten Bereiches, und einer negativen Beschleunigungsspanne, die zeitlich an die positive Beschleunigungsspanne angrenzt, mit einer Amplitude innerhalb eines spezifizierten Bereiches für eine Zeitdauer innerhalb eines spezifizierten Bereiches.

9. Verfahren nach Anspruch 1, das weiter Vergleichen der Beschleunigungsspannenwellenform mit einem oder mehreren vorbestimmten Mustern aufweist, die für einen Verkehrsflugzeugflugvorgang charakteristisch sind, wobei das Identifizieren des Verkehrsflugzeugbewegungsvorgangs weiter darauf basiert, ob eine minimale Anzahl von Flugvorgängen über eine gegebene Zeitperiode detektiert wird.

10. Verfahren nach Anspruch 1, das weiter Aktivieren oder Deaktivieren wenigstens einer Telefoniefunktion der Mobileinrichtung basierend auf der Verkehrsflugzeugbewegungsvorgangsidentifikation aufweist.

11. Verfahren nach Anspruch 1, wobei der eine oder die mehreren Beschleunigungsmesser in dem Maß kalibriert sind, dass jegliche Modifikation der Einrichtungsausrichtung eine weniger als einprozentige Veränderung der Beschleunigungsspanne generiert.

12. Eine Vorrichtung zum Identifizieren eines Verkehrsflugzeugbewegungsvorgangs an einer Mobileinrichtung, die Folgendes aufweist:
Mittel zum Bestimmen von skalaren Beschleunigungssignalen aus kalibrierten, triaxialen Beschleunigungsmesserdaten, die von einem oder mehrere Beschleunigungsmessern erhalten werden;
Mittel zum Filtern der skalaren Beschleunigungssignale, um Hochfrequenzrauschen zu verringern;
Mittel zum Verarbeiten der gefilterten, skalaren Beschleunigungssignale, um eine Beschleunigungsspannenwellenform zu generieren, wobei die Beschleunigungsspannenwellenform eine Differenz zwischen einem maximalen Wert und einem minimalen Wert der gefilterten, skalaren Beschleunigungssignale über ein Beobachtungsfenster hinweg nachverfolgt;
Mittel zum Vergleichen der Beschleunigungsspannenwellenform mit einem oder mehreren vorbestimmten Mustern, die für einen Verkehrsflugzeugbewegungsvorgang charakteristisch sind; und
Mittel zum Identifizieren eines Verkehrsflugzeugbewegungsvorganges basierend darauf, ob das Vergleichen zu einer wesentlichen Übereinstimmung führt.

13. Vorrichtung nach Anspruch 12, die weiter Folgendes aufweist:
einen oder mehrere Beschleunigungsmesser (110), die konfiguriert sind, um kalibrierte, triaxiale Beschleunigungsmesserdaten auszugeben; und
wobei die Mittel zum Bestimmen von skalaren Beschleunigungssignalen, die Mittel zum Filtern und die Mittel zum Verarbeiten
ein Beschleunigungsmerkmalextraktionselement (106) aufweisen; und wobei die Mittel zum Vergleichen und die Mittel zum Identifizieren einen Bewegungsvorgangidentifikationsprozessor (102) aufweisen.

14. Ein computerlesbares Medium, das Code aufweist, der, wenn er von einem Prozessor ausgeführt wird, den Prozessor veranlasst, das Verfahren nach einem der Ansprüche 1 bis 11 durchzuführen.

15. Ein System zum Identifizieren eines Verkehrsflugzeugbewegungsvorgangs an einer Mobileinrichtung, das Folgendes aufweist:
einen oder mehrere Prozessoren konfiguriert zum:
Bestimmen von skalaren Beschleunigungssignalen aus kalibrierten, triaxialen Beschleunigungsmesserdaten, die von einem oder mehrere Beschleunigungsmessern erhalten werden;
Filtern der skalaren Beschleunigungssignale, um Hochfrequenzrauschen zu verringern;
Verarbeiten der gefilterten, skalaren Beschleunigungssignale, um eine Beschleunigungsspannenwellenform zu generieren, wobei die Beschleunigungsspannenwellenform eine Differenz zwischen einem maximalen Wert und einem minimalen Wert der gefilterten, skalaren Beschleunigungssignale über ein Beobachtungsfenster hinweg nachverfolgt;
Vergleichen der Beschleunigungsspannenwellenform mit einem oder mehreren vorbestimmten Mustern, die für einen Verkehrsflugzeugbewegungsvorgang charakteristisch sind; und
Identifizieren eines Verkehrsflugzeugbewegungsvorganges basierend darauf, ob das Vergleichen zu einer wesentlichen Übereinstimmung führt; und
Speicher (108), der an den einen oder die mehreren Prozessoren gekoppelt ist und konfiguriert ist, um damit in Beziehung stehende Daten und/oder Instruktionen zu speichern.

## Revendications

1. Procédé pour identifier un événement de mouvement d'avion de ligne au niveau d'un dispositif mobile, comprenant :
déterminer (708) des signaux d'accélération scalaires à partir de données d'accéléromètre triaxiales étalonnées provenant d'un ou plusieurs accéléromètres ;
filtrer (712) les signaux d'accélération scalaires pour réduire le bruit à haute fréquence ;
traiter les signaux d'accélération scalaires filtrés pour générer (716) une forme d'onde d'écart d'accélération, la forme d'onde d'écart d'accélération suivant la différence entre une valeur maximum et une valeur minimum des signaux d'accélération scalaires filtrés sur une fenêtre d'observation ;
comparer (720) la forme d'onde d'écart d'accélération à un ou plusieurs motifs prédéterminés caractéristiques d'un événement de mouvement d'avion de ligne ; et
identifier un événement de mouvement d'avion de ligne sur la base du fait que la comparaison montre une concordance notable (728).

2. Procédé selon la revendication 1, dans lequel l'événement de mouvement d'avion de ligne est un événement de décollage ou un événement d'atterrissage.

3. Procédé selon la revendication 1, dans lequel la fenêtre d'observation a une période comprise entre environ dix et environ cent quatre-vingt secondes.

4. Procédé selon la revendication 1, dans lequel la détermination comprend le calcul d'une moyenne des données d'accéléromètre triaxiales étalonnées sur une période de moyenne comprise entre environ une et environ soixante secondes.

5. Procédé selon la revendication 1, dans lequel lesdits un ou plusieurs motifs prédéterminés comprennent un motif d'écart d'accélération ayant une période de repos suivie d'un événement de course au sol de décollage et de montée d'avion de ligne.

6. Procédé selon la revendication 5, dans lequel l'événement de course au sol de décollage et de montée d'avion de ligne est défini par un écart d'accélération positif ayant une amplitude supérieure à un seuil spécifié pendant une période de temps dans une plage spécifiée.

7. Procédé selon la revendication 1, dans lequel lesdits un ou plusieurs motifs prédéterminés comprennent un motif d'écart d'accélération ayant un événement de course au sol d'atterrissage suivi d'une période de repos.

8. Procédé selon la revendication 7, dans lequel l'événement de course au sol d'atterrissage est défini par un écart d'accélération positif ayant une amplitude dans une plage spécifiée pendant une période de temps dans une plage spécifiée, et un écart d'accélération négatif, adjacent temporellement à l'écart d'accélération positif, ayant une amplitude dans une plage spécifiée pendant une période temporelle dans une plage spécifiée.

9. Procédé selon la revendication 1, comprenant en outre la comparaison de la forme d'onde d'écart d'accélération à un ou plusieurs motifs prédéterminés caractéristiques d'un événement de vol d'avion de ligne, l'identification de l'événement de mouvement d'avion de ligne étant en outre basée sur le fait qu'un nombre minimum d'événements de vol sont détectés dans une période de temps donnée.

10. Procédé selon la revendication 1, comprenant en outre l'activation ou la désactivation d'au moins une fonction téléphonique du dispositif mobile sur la base de l'identification de l'événement de mouvement d'avion de ligne.

11. Procédé selon la revendication 1, dans lequel lesdits un ou plusieurs accéléromètres sont étalonnés dans la mesure où toute modification de l'orientation du dispositif génère moins de un pourcent de changement dans l'écart d'accélération.

12. Appareil pour identifier un événement de mouvement d'avion de ligne au niveau d'un dispositif mobile, comprenant :
des moyens pour déterminer des signaux d'accélération scalaires à partir de données d'accéléromètre triaxiales étalonnées provenant d'un ou plusieurs accéléromètres ;
des moyens pour filtrer les signaux d'accélération scalaires pour réduire le bruit à haute fréquence ;
des moyens pour traiter les signaux d'accélération scalaires filtrés pour générer une forme d'onde d'écart d'accélération, la forme d'onde d'écart d'accélération suivant la différence entre une valeur maximum et une valeur minimum des signaux d'accélération scalaires filtrés sur une fenêtre d'observation ;
des moyens pour comparer la forme d'onde d'écart d'accélération à un ou plusieurs motifs prédéterminés caractéristiques d'un événement de mouvement d'avion de ligne ; et
des moyens pour identifier un événement de mouvement d' avion de ligne sur la base du fait que la comparaison montre une concordance notable.

13. Appareil selon la revendication 12, comprenant en outre :
un ou plusieurs accéléromètres (110) agencés pour produire des données d'accéléromètre triaxiales étalonnées ; et
dans lequel les moyens pour déterminer des signaux d'accélération scalaires, les moyens pour filtrer, et les moyens pour traiter comprennent un extracteur de caractéristiques d'accélération (106) ; et dans lequel les moyens pour comparer et les moyens pour identifier comprennent un processeur d'identification d'événements de mouvement (102).

14. Support lisible par un ordinateur comprenant du code qui, lorsqu'il est exécuté par un processeur, amène le processeur à réaliser le procédé de l'une quelconque des revendications 1 à 11.

15. Système pour identifier un événement de mouvement d'avion de ligne au niveau d'un dispositif mobile, comprenant :
un ou plusieurs processeurs agencés pour :
déterminer des signaux d'accélération scalaires à partir de données d'accéléromètre triaxiales étalonnées provenant d'un ou plusieurs accéléromètres ;
filtrer les signaux d'accélération scalaires pour réduire le bruit à haute fréquence ;
traiter les signaux d'accélération scalaires filtrés pour générer une forme d'onde d'écart d'accélération, la forme d'onde d'écart d'accélération suivant la différence entre une valeur maximum et une valeur minimum des signaux d'accélération scalaires filtrés sur une fenêtre d'observation ;
comparer la forme d'onde d'écart d'accélération à un ou plusieurs motifs prédéterminés caractéristiques d'un événement de mouvement d'avion de ligne ; et
identifier un événement de mouvement d'avion de ligne sur la base du fait que la comparaison montre une concordance notable ; et
une mémoire (108) couplée auxdits un ou plusieurs processeurs et agencée pour mémoriser des données et/ou des instructions associées.
